# EUROPÄISCHE PATENTSCHRIFT

(11) **EP 1 318 405 B1**
(45) Veröffentlichungstag und Bekanntmachung des Hinweises auf die Patenterteilung: **17.11.2004**
(21) Anmeldenummer: 01128851.1
(22) Anmeldetag: 04.12.2001
(51) Int. Cl.: G01N 33/68, G01N 33/569

(54) **Verfahren zur Diagnose von Sepsis unter Bestimmung löslicher Cytokeratinfragmente**
Diagnosis of sepsis determining soluble cytokeratins
Diagnostic de la septicémie par détermination des cytokératines solubles

(43) Veröffentlichungstag der Anmeldung: 11.06.2003
(73) Patentinhaber: B.R.A.H.M.S Aktiengesellschaft, 16761 Hennigsdorf (DE)
(72) Erfinder: Bergmann, Andreas, Dr., 12351 Berlin (DE)
(74) Vertreter: Andrae, Steffen, Dr.

(56) Entgegenhaltungen:
- STREICHER JOHANNES ET AL: "Anticytokeratins are a potential source of false-positive indirect immunofluorescence assays for C-ANCA." JOURNAL OF CLINICAL LABORATORY ANALYSIS, Bd. 12, Nr. 1, 1998, Seiten 54-59, XP008003116 ISSN: 0887-8013
- TREVISANI LUCIO ET AL: "Cytokeratin tumor marker levels in bronchial washing in the diagnosis of lung cancer." CHEST, Bd. 109, Nr. 1, 1996, Seiten 104-108, XP008003364 ISSN: 0012-3692
- REINHART, K ET AL: "Intensivmedizin: Sepsis und septischer Schock (Seiten 756-760)" 2001 , THIEME VERLAG, XP008003130 * Seite 756, rechte Spalte, Absatz 3 *
- BEISHUIZEN A ET AL: "Endogenous mediators in sepsis and septic shock" ADVANCES IN CLINICAL CHEMISTRY, Bd. 33, 1999, Seiten 55-131, XP008003176

### SEQUENCE LISTING

<110> B.R.A.H.M.S Aktiengesellschaft
<120> Verfahren zur Diagnose von Sepsis unter Bestimmnug löslicher Cytokeratinfragmente
<130> 3636AS
<140>
   <141>
<160> 1
<170> PatentIn Ver. 2.1
<210> 1
   <211> 17
   <212> PRT
   <213> Artificial Sequence
<220>
   <223> Description of Artificial Sequence: Contains a partial (AA 14 to 29) sequence of cytokeratin 1
<400> 1
   Cys Leu Gln Gln Val Asp Thr Ser Thr Arg Thr His Asn Leu Glu Pro
     1               5                  10                  15
   Tyr

## Beschreibung

Die Erfindung betrifft ein neues Verfahren zur Sepsisdiagnose, bei dem oder im Rahmen dessen lösliche Cytokeratinfragmente bestimmt werden.

Die Erfindung beruht auf dem erstmaligen Nachweis stark erhöhter Konzentrationen von verschiedenen löslichen Cytokeratinfragmenten im Blutkreislauf von Patienten, bei denen aufgrund klinischer Befunde und gleichzeitig erhöhten Serumkonzentrationen des bekannten Sepsismarkers Procalcitonin eine bakterielle Sepsis diagnostiziert worden war. Zu den genannten löslichen Cytokeratinfragmenten gehören insbesondere auch die an sich aus anderen Zusammenhängen, und zwar als Tumormarker, bekannten Parameter CYFRA 21-1 sowie TPS (tissue polypeptide specific antigen) und dessen unspezifischerer historischer Vorläufer TPA (tissue polypeptide antigen).

Die vorliegende Erfindung hat ihren Ursprung in intensiven Forschungsarbeiten der Anmelderin im Zusammenhang mit weiteren Verbesserungen der Diagnose und Therapie von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Bei Sepsis bzw. dem septische Schock weiten sich entzündungsspezifische Reaktionskaskaden unkontrolliert auf den gesamten Körper aus und können dabei, im Sinne einer überschießenden systemischen Immunantwort, lebensbedrohlich werden. Zu den gegenwärtigen Kenntnissen über das Auftreten und die möglichen Rolle einzelner Gruppen endogener sepsisspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung wird daher der Begriff Sepsis in Anlehnung an die Definitionen verwendet, wie sie den genannten Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß oder vermutet, dass sie an einem Entzündungsgeschehen beteiligt sind, eignen sich dabei für Zwecke der klinischen Sepsisdiagnostik insbesondere solche, die bei Sepsis oder bestimmten Phasen einer Sepsis mit hoher Sensitivität und Spezifität auftreten bzw. deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen mit dem jeweiligen Biomarker im Vordergrund, ohne dass dessen Rolle in der komplexen Kaskade der am Sepsisgeschehen beteiligten endogenen Substanzen genau bekannt sein muss.

Eine bekannte, als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Besonders wertvoll ist die Procalcitoninbestimmung ferner zur therapiebegleitenden Verlaufsbeobachtung einer Sepsis. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig bei der Anmelderin intensive Anstrengungen unternommen, weitere Biomarker zu finden, die die Procaicitonin-Bestimmung ergänzen können und/oder zusätzliche Informationen für Zwecke der Feindiagnostik bzw. Differentialdiagnostik von septischen Erkrankungen zu liefern vermögen. So werden insbesondere weitere Biomarker für die Sepsisdiagnostik gesucht, deren Spiegel in biologischen Flüssigkeiten, insbesoneder in Seren, zwar regelmäßig erhöht sind, die bei ihrer Bestimmung jedoch nicht einfach die Ergebnisse der Procalcitoninbestimmung duplizieren, sondern zusätzliche Informationen liefern, und zwar insbesondere zum Stadium des Sepsisgeschehens, d.h. eine dem Sepsisverlauf eher zeitlich zuzuordnende Information, und/oder zum Ausgangs- oder Schwerpunktorgan eines septischen Geschehens, d.h. eine lokalisierende Information. Ziel ist letztlich die Selektion eines Satzes von Sepsisparametern, die in biologischen Flüssigkeiten, d.h. insbesondere in Serum, aber z.B auch in anderen biologischen Flüssigkeiten wie Urin, von Sepsispatienten bzw. potentiellen Sepsispatienten gleichzeitig bestimmt werden, z.B. unter Nutzung der sog. Chip-Technologie oder immunchromatographischer Verfahren ("Point of Care" oder POC-Bestimmungen), und dabei in ihrer Gesamtheit ein Informationsmuster liefern, das den Informationswert der Bestimmung nur eines einzelnen Parameters klar übertrifft.

Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Da die bei Sepsis erhöhten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Sepsisgeschehen einzugreifen, um zum Beispiel die bei Sepsis beobachtete systemische Ausbreitung der Entzündung möglichst frühzeitig zu stoppen. In diesem Sinne sind nachweislich am Sepsisgeschehen beteiligte endogene Substanzen auch als potentielle therapeutische Targets anzusehen.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin signifikant erhöht ist, sondern auch für andere Substanzen, die zu den Peptid-Prohormonen gerechnet werden können, signifikant erhöhte Konzentrationen beobachtet werden können. Es sind in diesem Zusammenhang zu nennen die Peptid-Prohormone pro-Enkephalin, pro-Gastrin-Releasing Peptid (proGRP), pro-Endothelin-1, pro-Brain-Natriuretic-Peptid (pro-BNP), pro-Atrial-Natriuretic-Peptid (pro-ANP), pro-Leptin, pro-Neuropeptid-Y, pro-Somatostatin, pro-Neuropeptid-YY, pro-Interleukin 6 oder pro-Interleukin-10. Während das beschriebene Phänomen gut dokumentiert ist, sind die Ursachen für den Anstieg der Konzentrationen von Prohormonen bei Sepsis weiterhin weitgehend ungeklärt.

In der älteren Anmeldung DE 101 30 985.6 werden ferner Ergebnisse eines rein empirischen Versuchsansatzes beschrieben, bei dem nach einer experimentell durch Toxinverabreichung (LPS aus Salmonella Typhimurium) bei Pavianen ausgelösten künstlichen Sepsis in Leberproben unter anderem ein Peptid gefunden wurde, das nur bei den behandelten Tieren nachweisbar war. Es erwies sich als ein lösliches Cytokeratin-1-Fragment (nachfolgend abgekürzt sCY1F). Wie im experimentellen Teil dieser Anmeldung gezeigt wird, wird dieses zuerst in Leberextrakten von Pavianen gefundene Fragment sCY1F mit einer Sensitivität von 95% auch in Seren von humanen Sepsispatienten gefunden, während es in Seren Gesunder nicht nachweisbar ist.

Diese experimentellen Befunde ließen es als lohnend erscheinen, experimentell zu prüfen, ob in den Seren von Sepsispatienten auch erhöhte Konzentrationen anderer löslicher Cytokeratinfragmente gemessen werden können, zum Beispiel von solchen löslichen Cytokeratinfragmenten, die in der medizinischen Diagnostik bereits als Tumormarker eine Rolle spielen.

Überraschenderweise zeigte sich, dass bei Sepsis auch einige bisher als typische Tumormarker angesehene lösliche Cytokeratinfragmente signifikant erhöht sind. Das deutet darauf hin, dass derartige Fragmente nicht tumorspezifisch gebildet werden, sondern eher ein generalisiertes physiologisches Krisengeschehen anzeigen, das auch Gewebe bzw. Organe erfaßt, die diese Tumormarker freisetzen. Und obwohl, wie in dieser Anmeldung und gleichzeitig eingereichten weiteren Anmeldungen gezeigt wird, die Konzentrationen der löslichen Cytokeratinfragmente und einiger anderer Tumormarker bei Sepsis mit hoher Sensitivität erhöht sind, besteht gleichzeitig keine quantitative Korrelation der gemessenen Werte zu den ebenfalls signifikant erhöhten Procalcitoninkonzentrationen, d.h. bei einzelnen Patienten werden zwar beide Parameter erhöht gefunden, jedoch teilweise in ganz unterschiedlichen Relativmengen.

Die vorliegende Erfindung beruht auf dem erstmaligen Nachweis, dass in biologischen Flüssigkeiten, insbesondere in Seren, von Sepsispatienten signifikant erhöhte physiologische Konzentrationen von löslichen Cytokeratinfragmenten gefunden werden, was diese, insbesondere in Kombination mit der Bestimmung weiterer Sepsisparameter, für die Sepsisdiagnostik geeignet macht.

Das erfindungsgemäße Verfahren und bestimmte bevorzugte Ausgestaltungen davon sind in den Ansprüchen 1 bis 6 genauer definiert.

Es war bisher nicht bekannt, dass die Konzentrationen von löslichen Cytokeratinfragmenten in biologischen Flüssigkeiten, insbesondere in Seren, bei Sepsis signifikant erhöht sind und dass daher eine Bestimmung der Konzentration von löslichen Cytokeratinfragmenten auch für die Sepsisdiagnostik von Bedeutung sein kann. Das gilt trotz der Tatsache, dass einige lösliche Cytokeratinfragmente, und zwar insbesondere in Form des Parameters CYFRA 21-1 sowie des Parameters TPS bzw. seines weniger spezifischen Vorläufers TPA, bereits im Rahmen der Tumordiagnostik bestimmt werden und zur Bestimmung dieser Parameter geeignete Assays kommerziell erhältlich sind. In Streicher, J. et al. (J.o. Clin. Lab. Analysis, Vol. 12, S. 54-59, 1998) wird gezeigt, daß Autoantikörper gegen bestimmte Cytokeratine in Sepsis-Patienten erhöht sein können.

Aufgrund der vorliegenden Erfindung ist es möglich, die Bestimmung von löslichen Cytokeratinfragmenten auch im Rahmen eines diagnostischen Sepsis-Nachweisverfahrens zu nutzen. Von besonderem Interesse ist die Eignung von löslichen Cytokeratinfragmenten als Prognosemarker und Marker für die Überwachung der Krankheitsverlaufs von Sepsis, insbesondere im Rahmen einer Kombinationsmessung mit anderen Markern. Aufgrund der hohen Sensitivität und Spezifität eignen sich lösliche Cytokeratinfragmente, einzeln oder in einer Kombination verschiedener Typen, auch als Marker, die im Rahmen einer Sepsis-Erstdiagnose bestimmt werden können.

Neben einer Kombination mit einer Procalcitoninmessung kommt insbesondere eine Kombination der Messung von einem oder mehreren löslichen Cytokeratinfragment(en) mit der Bestimmung anderer Marker für Sepsis und systemische Entzündungen, die bisher als typische Tumormarker angesehen wurden, in Betracht, und zwar insbesondere mit CA 19-9, CA 125, S100B oder an der Entzündungsregulation beteiligten S100A-Proteinen oder mit der Bestimmung der in den älteren, nachfolgend genannten deutschen Patentanmeldungen der Anmelderin beschriebenen neuartigen Sepsismarker Inflammin (DE 101 19 804.3) und CHP (DE 101 31 922.3) und/oder eines oder mehrerer der o.g. Prohormone. Auch eine gleichzeitige Bestimmung des bekannten Entzündungsparameters C-reaktives Protein (CRP) kann vorgesehen sein. Aufgrund der in dieser und den parallelen Anmeldungen beschriebenen neuen Ergebnisse ist für die Sepsis-Feindiagnose außerdem generell eine Kombination mit Messungen von bekannten oder noch aufzufindenden Biomolekülen in Betracht zu ziehen, die als gewebe- bzw. organspezifische Entzündungsmarker geeignet sind.

Als CYFRA 21-1 wird ein lösliches Fragment des Cytokeratins 19 bezeichnet. Obwohl Cytokeratin 19 weder als organ- noch als tumorspezifisches Protein angesehen werden kann, spielt die Bestimmung von CYFRA 21-1 eine wichtige Rolle als Tumormarker bei der Diagnostik, Therapie- und Verlaufskontrolle insbesondere des Bronchialkarzinoms und zur Verlaufskontrolle des Blasenkarzinoms (vgl. Lothar Thomas (Hrsg.): Labor und Diagnose, Kapitel 34.10, S.987-992, 5.Auflage, 1998 TH-Books Verlagsgesellschaft).

Nach den bisherigen Erkenntnissen kann CYFRA 21-1 auch bei einigen nicht-malignen Erkrankungen erhöht sein, wobei jedoch Werte von >10 ng/ml nur in äußerst seltenen Fällen mit einer benignen Erkrankung vereinbar sind. Bei benignen Erkrankungen wurden die höchsten Werte in Fällen gastrointestinaler Erkrankungen gemessen. Zur Messung von CYFRA 21-1 und zur Interpretation der Messergebnisse kann ergänzend zu Thomas, a.a.O. und den darin angeführten Quellen z.B. verwiesen werden auf M. Sarwar et al., Ann Nucl Med Vol.8, No.4, 301-306, 1994; W. Ebert et al., Scand J Clin Invest 1995; 55 Suppl 221:72-80; Young-Chul Kim et al., Lung Cancer 30 (2000) 187-192.

Als TPS (tissue polypeptide specific antigen) wird ein Cytokeratin-18-Fragment bezeichnet, das mit dem seit mehr als 40 Jahren bekannten sogenannten TPA (tissue polypeptide antigen) verwandt ist, jedoch besser charakterisiert und spezifischer ist. Es kann diesbezüglich verwiesen werden auf Lars Rylander et al., Eur.J.Biochem. 241, 309-314, (1996); und R. Einarsson et al., Anticancer Research 17:3121-3124 (1997). Ein zu seiner Bestimmung verwendbarer Antikörper M3 bindet an die Aminosäuren 322-340 des carboxyterminalen Abschnitts des Cytokeratins 18. Die Eignung und Verwendung von TPS als Tumormarker für Tumoren wie das Hepatozelluläre Karzinom, Brustkrebs und Prostatakrebs wird diskutiert z.B. in Wei-Jen Yao et al., Oncology 2001; 61:64-70; M.Sütterlin et al., Anticancer research 17:2963-2966 (1997); J.M.Wolff et al., Anticancer Research 20:5003-5006 (2000).

In den Arbeiten von M.Sütterlin et al., Anticancer Research 17:2963-2966 (1997); B.Nisman et al., Cancer 1998; 82:1850-1859; und V.Stearns et al., Breast Cancer Research and Treatment 52:239-259, 1998 wird die Eignung von TPS als Marker für organspezifische Tumoren verglichen mit anderen Tumormarkern wie dem weiteren Cytokeratinfragment-Marker CYFRA 8/18, CYFRA 21-1 und anderen klassischen tumorassoziierten Antigenen und CEA.

Bei septischen Patienten wurden nach diesseitiger Kenntnis noch keine systematischen Messungen der löslichen Cytokeratinfragmente wie CYFRA 21-1 oder TPS durchgeführt. Über in der großen Mehrzahl der Fälle signifikant erhöhte Messwerten der als typische Tumormarker angesehenen Cytokeratinfragmente bei Patienten mit systemischen Entzündungen (Sepsis) wurde noch nichts bekannt.

Eine deutliche Erhöhung der Konzentrationen der löslichen Cytokeratinfragmente CYFRA 21-1 und TPS sowie des neuen Entzündungsmarkers sCYF1F in den Seren der überwiegenden Zahl von Sepsispatienten wurde, die in der älteren Patentanmeldung DE 101 30 985.6 vorgestellten Befunde der Modellverscuhe mit Pavianen ergänzend, erstmals bei den von der Anmelderin durchgeführten Untersuchungen festgestellt, die in den nachfolgenden Versuchsberichten unter Bezugnahme auf vier Figuren beschrieben werden.

In den Figuren zeigen:
- Fig.1: die Ergebnisse der Bestimmung des Parameters CYFRA 21-1 (ein lösliches Cytokeratin-19-Fragment) in den Seren von 169 Sepsispatienten im Vergleich mit einer Gruppe von 50 Kontrollpersonen (Blutspendern);
- Fig.2: die quantitative Korrelation der Ergebnisse der CYFRA 21-1-Bestimmungen der 169 Sepsispatienten von Fig.1 mit den Ergebnissen der Procalcitoninbestimmung in den gleichen Seren;
- Fig.3: die Ergebnisse der Bestimmung des Parameters TPS (tissue polypeptide specific antigen; ein lösliches Cytokeratin-18-Fragment) in den Seren von 49 Sepsispatienten im Vergleich mit einer Gruppe von 21 Kontrollpersonen (Blutspendern);
- Fig.4: die Ergebnisse der Bestimmung des Parameters sCY1F (gemäss DE 101 30 985.6; ein lösliches Cytokeratin-1-Fragment) in den Seren von 20 Sepsispatienten im Vergleich mit einer Gruppe von 16 Kontrollpersonen (Blutspendern).

### Versuchsberichte:

### 1. Bestimmung von CYFRA 21-1

In 169 Seren von Sepsis-Patienten, bei denen hohe Werte für den Sepsismarker Procalcitonin (PCT) gefunden worden waren, wurden unter Verwendung eines kommerziellen Assays (KRYPTOR-CYFRA 21-1 der B.R.A.H.M.S Diagnostica GmbH) die Serumkonzentrationen des Tumormarkers CYFRA 21-1 bestimmt. Bei 81 % der Sepsisseren wurden erhöhte CYFRA 21-1 Konzentrationen (mehr als 1 ng/ml) gefunden. Bei den 50 zur Kontrolle verwendeten Seren überschritten nur in zwei Fällen die CYFRA 21-1 Konzentrationen ganz geringfügig den Wert von 1 ng/ml.

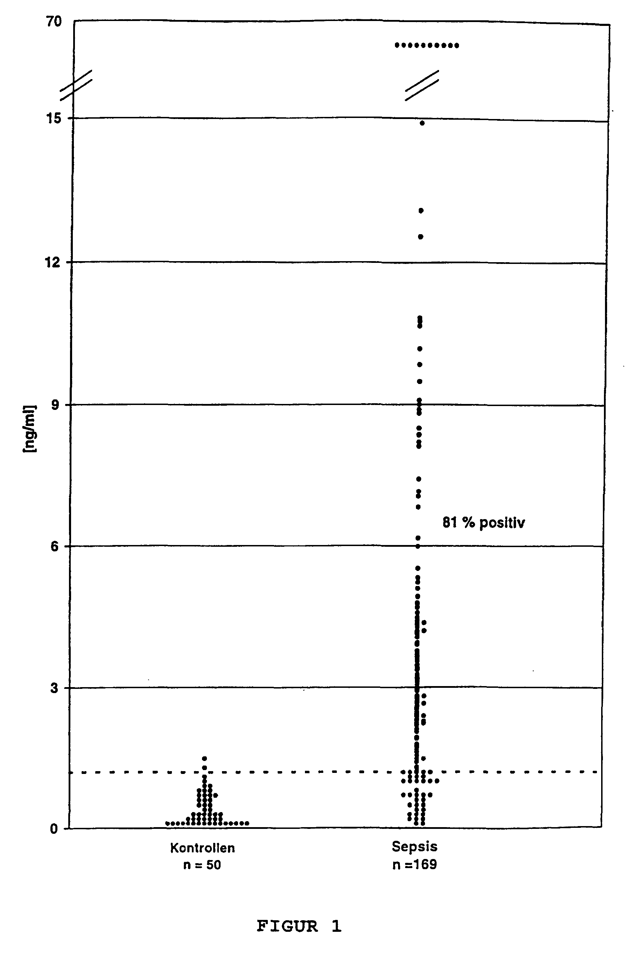

Eine graphische Darstellung der Messergebnisse ist in Figur 1 gezeigt.

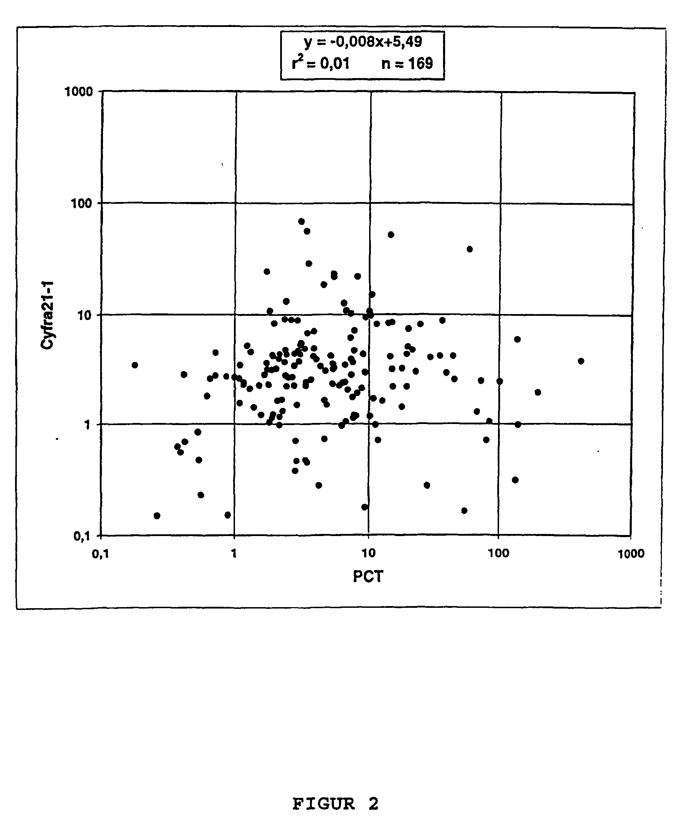

Werden die für individuelle Seren gemessenen CYFRA 21-1-Werte den für PCT gemessenen Werten gegenübergestellt, ergibt sich keine positive quantitative Korrelation in dem Sinne, dass in Seren, in denen die höchsten PCT Konzentrationen gefunden wurden, auch die höchsten CYFRA 21-1-Werte gefunden werden. Figur 2 zeigt die bei einer solchen Gegenüberstellung gefundenen Korrelationen. Es ist zu erkennen, dass hohe CYFRA 21-1-Werte (oberes Drittel des Diagramms) auch bei mäßigen PCT-Konzentrationen erhalten werden, und bei sehr hohen PCT-Konzentrationen (rechtes Drittel des Diagramms) mäßige Werte für CYFRA 21-1.

### 2. Bestimmung von TPS

In 49 Seren von Sepsis-Patienten, bei denen hohe Werte für den Sepsismarker Procalcitonin (PCT) gefunden worden waren, wurden unter Verwendung eines kommerziellen Assays zur Bestimmung von TPS (TPS™ der Fa. DPC-BIERMANN, Bad Nauheim) die Serumkonzentrationen des Tumormarkers TPS bestimmt. Bei 90 % der Sepsisseren wurden stark erhöhte TPS-Konzentrationen (mehr als 66 U/l) gefunden, während gleichzeitig alle 21 Kontrollseren (Seren gesunder Blutspender) negativ waren, d.h. Messwerte unter dem Bezugswert von 66 U/l aufwiesen.

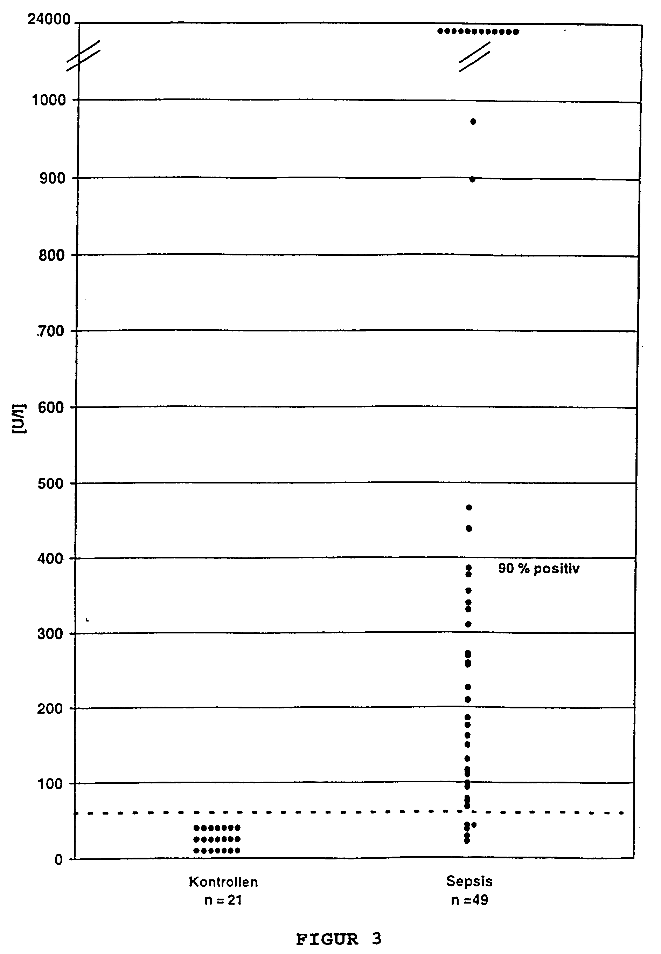

Eine graphische Darstellung der Ergebnisse der TPS-Messungen ist in Figur 3 gezeigt.

### 3. Bestimmung von sCY1F

In 20 Seren von Sepsis-Patienten, bei denen hohe Werte für den Sepsismarker Procalcitonin (PCT) gefunden worden waren, wurden die Serumkonzentrationen von löslichen Cytokeratin-1-fragmenten sCY1F gemäß DE 101 30 985.6 bestimmt. Bei 95 % der Sepsisseren wurden stark erhöhte sCY1F-Konzentrationen (mehr als 3 ng/ml) gefunden.

Für die orientierenden Bestimmungen von sCY1F in Sepsisseren wurde ein speziell dafür entwickelter kompetitiver Lumineszenz-Immunoassay eingesetzt, bei dem Schaf-Antikörper gegen ein Peptid, das eine Teilsequenz des Cytokeratin-1-Fragments, die die Aminosäuren 214 bis 229 der SEQ ID NO:3 aus DE 101 30 985.6 umfasste, verwendet wurden. Das zur Antikörpergewinnung und als Kompetitor verwendete synthetische Peptid ist unter der Bezeichnung Peptid PLY17 kommerziell erhältlich (Jerini BioTools GmbH). Es weist die im Sequenzprotokoll der vorliegenden Anmeldung gezeigte Aminosäuresequenz SEQ ID NO:1 auf.

Zur Durchführung der Bestimmungen von sCY1F wurde wie folgt vorgegangen:

Poylstyrol-Röhrchen (Fa. Greiner) wurden mit 100 ng Peptid (PLY17; SEQ ID NO:1) in 300 µl PBS beschichtet. Nach einer 20-stündigen Inkubation bei Raumtemperatur wurde mit 2 x 4 ml PBS, enthaltend 1% BSA, gewaschen. Die peptidbeschichteten Röhrchen wurden dann als Festphase für die Durchführung der nachfolgenden Messungen eingesetzt, bei denen die immobilisierten Peptide und die Cytokeratin-1-Fragmente aus der Probe um einen in Form eines Antiserums zugesetzten Schaf-Antikörpers gegen die o.g. Teilpeptidsequenz konkurrierten.

Zur Messung wurde nach dem folgenden Schema vorgegangen:
1. Pipettiere in die o.g. Röhrchen 100 µg der Probe (Sepsisserum oder Kontrollserum bzw. Kalibratorlösung);
2. Pipettiere 200 µl Antiserum (1:5000 mit PBS verdünnt);
3. Inkubiere 2 h bei Raumtemperatur unter Schütteln;
4. Wasche den ungebundenen Antikörper aus dem Röhrchen (4 x mit 1 ml PBS befüllt und dekantiert);
5. Gebe zur Markierung der festphasen-gebundenen Antikörper einen mit einem Akridiniumester markierten Eselanti-Schaf-Antikörper (B.R.A.H.M.S Diagnostica) in 300 ml PBS, 1 %BSA zu;
6. Entferne nach 2 h Inkubation bei Raumtemperatur den ungebundenen Markierungsantikörper, wasche wie unter 4.;
7. Vermesse den an die Festphase gebundenen Akridiniumester auf bekannte Weise mittels eines Luminometers (Fa. Berthold).

Zur Erstellung eines Eichkurve wurden Lösungen mit bekannten Mengen des synthetischen Peptids (SEQ ID NO:1) eingesetzt, und die Konzentrationen des Cytokeratin-1-Fragments wurden durch Vergleich der Messwerte für die Sepsisseren mit der Eichkurve bestimmt.

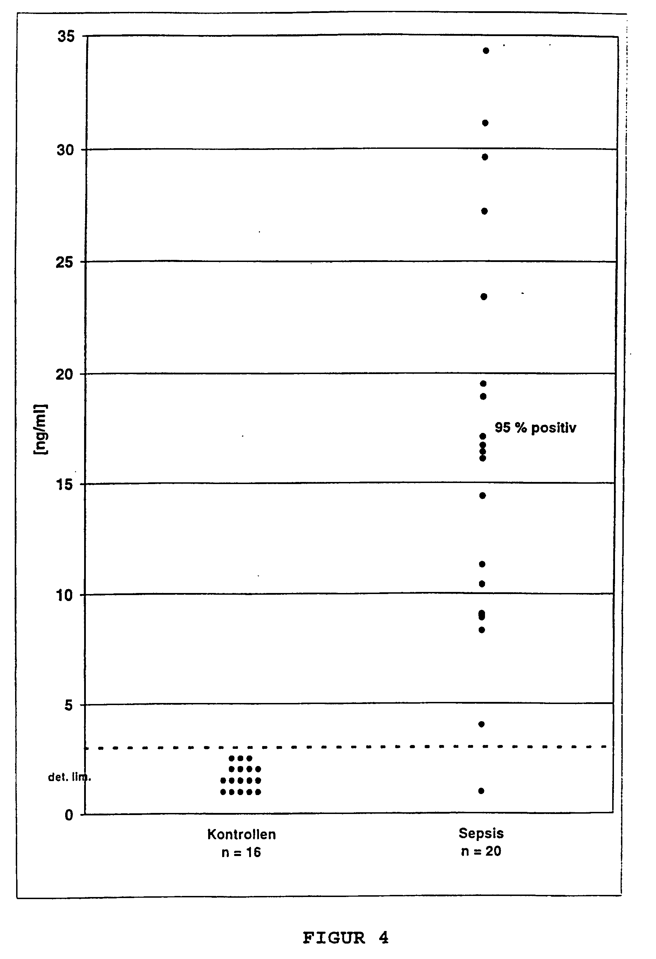

Eine graphische Darstellung der Messergebnisse ist in Figur 4 gezeigt. Bereits mit dem beschriebenen provisorischen, recht einfachen und unempfindlichen kompetitiven Messverfahren wird eine sehr gute Sensitivität der Bestimmung der Cytokeratin-1-Fragmente bei Sepsis ersichtlich.

Die weitgehende Unabhängigkeit der quantitativen Ergebnisse der Bestimmung von löslichen Cytokeratinfragmenten von denen der PCT-Bestimmung zeigt, dass trotz der für beide Parameter bei Sepsis erhöhten Werte offensichtlich unterschiedliche Effekte erfasst werden, was bedeutet, dass die Messung beider Parameter mehr Informationen liefert als die Messung nur eines der Parameter.

Die Kombination der Bestimmung von löslichen Cytokeratinfragmenten mit der eines oder mehrerer anderer Sepsismarker bietet sich daher zur Verbesserung der Feindiagnostik von Sepsis und zur Verbesserung der Prognose des Krankheitsverlaufs und zur therapiebegleitenden Verlaufskontrolle bei Sepsispatienten an, wobei die klare Hoffnung besteht, dass die Interpretation der Ergebnisse solcher kombinatorischer Bestimmungen auf der Basis der genauen Auswertung einzelner möglichst vollständig dokumentierter Fälle (mit z.B. Angaben zur Art der Infektion, Grund und Verlauf der Sepsiserkrankung, Kenndaten zum Alter und Geschlecht der Patienten) mit der Anzahl der ausgewerteten Fälle stetig verbessert werden kann.

Die Bestimmung von löslichen Cytokeratinfragmenten kann dabei nach irgendeinem beliebigen geeigneten Nachweisverfahren erfolgen, wobei jedoch die Bestimmung in einer Patienten-Körperflüssigkeit, vor allem in einem Serum, aber auch z.B. im Urin, auf immundiagnostischem Wege unter Verwendung geeigneter selektiver Antikörper unter praktischen Gesichtspunkten am vorteilhaftesten erscheint. Es stehen bereits kommerzielle Assays zur Bestimmung von einigen löslichen Cytokeratinfragmenten zur Verfügung, die auch im Rahmen der vorliegenden Erfindung genutzt werden können. Dabei ist gegebenenfalls auf eine gute Meßgenauigkeit im für die Sepsisdiagnostik relevanten Messbereich zu achten.

Somit kann die Bestimmung von löslichen Cytokeratinfragmenten zur Sepsisdiagnose, und zwar insbesondere zur differentialdiagnostischen Früherkennung und zur Erkennung sowie für die Erstellung einer Verlaufsprognose, zur Beurteilung des Schweregrads und zur therapiebegleitenden Verlaufsbeurteilung von Sepsis erfolgen, und zwar indem man bei einem derartigen Verfahren in einer Probe einer biologischen Flüssigkeit eines Patienten den Gehalt von löslichen Cytokeratinfragmenten bestimmt und aus der festgestellten Anwesenheit und/oder Menge von löslichen Cytokeratinfragmenten auf das Vorliegen einer Sepsis schließt und das erhaltene Ergebnis mit dem Schweregrad, dem Fortschritt der Sepsis und/oder dem am stärksten von der Sepsis betroffenen Gewebe oder Organ korreliert und die Behandlungsmöglichkeiten entsprechend wählt und/oder die Behandlungsaussichten abschätzt.

## Patentansprüche

1. Verfahren zur differentialdiagnostischen Früherkennung und Erkennung, für die Verlaufsprognose und die Beurteilung des Schweregrads und zur therapiebegleitenden Verlaufsbeurteilung von Sepsis *in vitro,* **dadurch gekennzeichnet, dass** man in einer biologischen Flüssigkeit eines Patienten, bei dem eine Sepsis vorliegt oder ein Verdacht auf Sepsis besteht, die Menge eines oder mehrerer löslicher Cytokeratinfragmente bestimmt, die ausgewählt sind aus TPA, TPS und CYFRA 21-1, und aus der bestimmten Menge des bestimmten löslichen Cytokeratinfragments Schlüsse hinsichtlich des Vorliegens, des zu erwartenden Verlaufs, des Schweregrads und/oder des Erfolgs eingeleiteter Maßnahmen zur Therapie der Sepsis zieht.

2. Verfahren nach Anspruch 1, **dadurch gekennzeichnet, dass** die Bestimmung der löslichen Cytokeratinfragmente mittels eines immundiagnostischen Bestimmungsverfahrens erfolgt.

3. Verfahren nach einem der Ansprüche 1 oder 2, **dadurch gekennzeichnet, dass** es im Rahmen einer Multiparameter-Bestimmung durchgeführt wird, bei der gleichzeitig mindestens ein weiterer Sepsisparameter bestimmt wird und ein Messergebnis in Form eines Satzes von mindestens zwei Messgrößen gewonnen wird, der zur Sepsis-Feindiagnostik ausgewertet wird.

4. Verfahren nach Anspruch 3, **dadurch gekennzeichnet, dass** im Rahmen der Multiparameter-Bestimmung neben wenigstens einem löslichen Cytokeratinfragment wenigstens ein weiterer Parameter bestimmt wird, der ausgewählt ist aus der Gruppe, die besteht aus Procalcitonin, CA 125, CA 19-9, S100B, S100A-Proteinen, den Peptiden Inflammin und CHP, Peptid-Prohormonen und dem C-reaktiven Protein (CRP).

5. Verfahren nach Anspruch 3 oder 4, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

6. Verfahren nach Anspruch 5, **dadurch gekennzeichnet, dass** die Auswertung des mit der Messvorrichtung gewonnenen komplexen Messergebnisses mit Hilfe eines Computerprogramms erfolgt.

## Claims

1. Method for early differential diagnosis and detection, for prognosis and for assessing the severity and for therapy-accompanying assessment of the course of sepsis *in vitro*, **characterized in that** the amount of one or more soluble cytokeratin fragments selected from TPA, TPS and CYFRA 21-1 in a biological fluid of a patient in whom a sepsis is present or sepsis is suspected is determined and conclusions with regard to the presence, the expected course, the severity and/or the success of initiated measures for the therapy of the sepsis are drawn from the determined amount of the determined soluble cytokeratin fragment.

2. Method according to Claim 1, **characterized in that** the determination of said soluble cytokeratin fragments is effected by means of an immunodiagnostic assay method.

3. Method according to any of Claims 1 or 2, **characterized in that** it is carried out in the course of a multiparameter determination in which at least one further sepsis parameter is simultaneously determined and a measured result in the form of a set of at least two measured variables is obtained and is evaluated for fine sepsis diagnosis.

4. Method according to Claim 3, **characterized in that**, in the course of the multiparameter determination, at least one further parameter which is selected from the group consisting of procalcitonin, CA 125, CA 19-9, S100B, S100A proteins, the peptides inflammin and CHP, peptide prohormones and the C-reactive protein (CRP) is determined in addition to at least one soluble cytokeratin fragment.

5. Method according to Claim 3 or 4, **characterized in that** the multiparameter determination is carried out as a simultaneous determination by means of a chip technology measuring device or of an immunochromatographic measuring device.

6. Method according to Claim 5, **characterized in that** the evaluation of the complex measured result obtained using the measuring device is carried out with the aid of a computer program.

## Revendications

1. Procédé *in vitro* de détection précoce par diagnostic différentiel et de détection, de pronostic de l'évolution, de l'évaluation du degré de gravité et de l'évaluation de l'évolution en accompagnement d'une thérapie, d'une sepsie, **caractérisé en ce que** dans un liquide biologique d'un patient atteint de sepsie ou chez lequel on soupçonne une sepsie, on détermine la quantité d'un ou de plusieurs fragments solubles de cytokératine qui sont sélectionnés parmi le TPA, le TPS et le CYFRA 21-1, et dans lequel, à partir de la quantité déterminée du fragment soluble de cytokératine que l'on détermine, on tire des conclusions sur la présence, l'évolution attendue, le degré de gravité et/ou le résultat des mesures thérapeutiques de la sepsie que l'on a prises.

2. Procédé selon la revendication 1, **caractérisé en ce que** la détermination des fragments solubles de cytokératine s'effectue au moyen d'un procédé de détermination par immunodiagnostic.

3. Procédé selon l'une des revendications 1 ou 2, **caractérisé en ce qu'**il est exécuté dans le cadre d'une détermination de plusieurs paramètres dans lequel on détermine en même temps au moins un autre paramètre de sepsie et dans lequel on recueille un résultat de mesure qui présente la forme d'un ensemble constitué d'au moins deux grandeurs de mesure et évalué en vue d'un diagnostic fin de la sepsie.

4. Procédé selon la revendication 3, **caractérisé en ce que** dans le cadre de la détermination de plusieurs paramètres, en plus d'au moins un fragment soluble de cytokératine, on détermine au moins un autre paramètre qui est sélectionné dans le groupe qui est constitué de la procalcitonine, du CA 125, du CA 19-9, du S100G, des protéines S100A, des peptides inflammine et CHP, des peptides pro-hormones et de la protéine C-réactive (CRP).

5. Procédé selon les revendications 3 ou 4, **caractérisé en ce que** la détermination de plusieurs paramètres est réalisée sous la forme d'une détermination simultanée au moyen d'un dispositif de mesure basé sur une technologie à puce ou d'un dispositif de mesure par immunochromatographie.

6. Procédé selon la revendication 5, **caractérisé en ce que** l'évaluation du résultat complexe de mesure obtenue avec le dispositif de mesure s'effectue à l'aide d'un programme informatique.
